# FASCICULE DE BREVET EUROPEEN

(11) **EP 1 068 843 B1**
(45) Date de publication et mention de la délivrance du brevet: **17.11.2004**
(21) Numéro de dépôt: 00420083.8
(22) Date de dépôt: 26.04.2000
(51) Int. Cl.: A61F 2/34

(54) **Cotyle pour prothèse de hanche**
Gelenkpfanne für eine Hüftprothese
Acetabular cup for hip prosthesis

(30) Priorité: 13.07.1999 FR 9909303
(43) Date de publication de la demande: 17.01.2001
(73) Titulaire: Biomet France, 26000 Valence (FR)
(72) Inventeur: Bost, Joel, 69640 Jarnioux (FR); Braud, Gérard, 01000 Bourg en Bresse (FR); Brayer, Jean-Charles, Dr., 06950 Falicon (FR); Charpenet, Rémy, 88150 Thaon Les Vosges (FR); Darman, Zulgael, 88270 Hennecourt (FR); Ginefri, Jean-Paul, 22121 Fontaine Les Dijon (FR); Lamy, Henri, Dr., 39100 Dole (FR); Lantuejoul, Jean-Pierre, Dr., 38330 Saint Ismier (FR); Leroy, Jean-Michel, 74200 Anthy sur Leman (FR); Machet, Pierre, 74700 Sallanches (FR); Montbarbon, Eric, Dr., 73000 Barberaz (FR); Paillot, Jean-Michel, Dr., 73100 Tresserve (FR); Paulin, Marc, 52000 Chaumont (FR); Revel, Jean-Jacques, Dr., 07300 Saint Jean de Muzols (FR); Saragaglia, Dominique, 38170 Seyssinet Pariset (FR); Van Nieuwenhuyse, Nicolas, Dr., 74250 Viuz en Lallaz (FR); Benedetto, Muriel, 26000 Valence (FR); De Witte, Gérard, 26300 Chateauneuf sur Isere (FR)
(74) Mandataire: Vuillermoz, Bruno

(56) Documents cités:
- EP-A- 0 900 552
- WO-A-95/16413
- DE-A- 4 437 479
- FR-A- 2 715 556
- FR-A- 2 719 761

## Description

L'invention concerne un cotyle pour prothèse totale de hanche.

De manière connue, une prothèse de hanche comprend fondamentalement deux parties, respectivement un élément fémoral ou tige, destiné à être inséré dans le canal médullaire du fémur à prothéser, et, portant à son extrémité libre une tête sphérique, et un cotyle, mis en place au niveau de la cavité cotyloïdienne de l'aile iliaque de l'articulation considérée, ce cotyle étant destiné à recevoir la tête sphérique dudit élément fémoral.

Plus précisément, ce cotyle était réalisé en deux parties distinctes, à savoir respectivement :
■ une cupule dite de « soutien », destinée à être insérée dans la cavité cotyloïdienne de l'aile iliaque ;
■ et un noyau ou insert, dit de frottement, destiné à s'insérer dans la cupule de soutien, et dont la face interne de forme hémisphérique est destinée à recevoir la tête sphérique de l'élément fémoral.

En pratique, la cupule de soutien est rigide et est réalisée le plus souvent en métal, tel que par exemple en un alliage de titane.

En revanche, l'insert est le plus généralement réalisé en matière plastique, telle que notamment en polyéthylène haute densité, voire également en matière beaucoup plus dure, telle qu'en céramique, et notamment en alumine.

Dans le cadre de l'utilisation de tels cotyles, l'un des problèmes fondamentaux auxquels se heurtent les praticiens, relève de l'ancrage ferme et irréversible de la cupule de soutien au sein de la cavité cotyloïdienne de l'aile iliaque.

Par ailleurs, on souhaite de plus en plus, lors de la pose de ces cupules, s'affranchir de la mise en oeuvre de ciment afin d'une part, d'éviter toute réaction de l'organisme vis à vis de ce dernier, et d'autre part, de faciliter la mise en place desdites cupules.

Il a ainsi été proposé des cupules de soutien, dont la face convexe externe présente des fentes d'expansion s'étendant selon des méridiens, ouvertes au niveau de l'équateur. De telles cupules dites d'expansion, fixées au moyen de vis ou analogues, permettent certes de s'affranchir du ciment, mais ne donnent pas encore satisfaction sur le plan de la fixation définitive, et notamment au niveau de la fixation secondaire liée à la repousse osseuse.

Il a également été proposé une cupule également rigide, dont partie de la surface externe est munie de cannelures usinées dans la masse, et s'étendant environ sur le tiers ou la moitié de la hauteur de ladite cupule (FR-A-2 681 238).

Si certes, par ce biais, on aboutit à une fixation améliorée de la cupule au sein de la cavité cotyloïdienne, la mise en oeuvre de ces cannelures génère une sur-épaisseur, rendant plus délicate la pose de ladite cupule, et en outre, altérant la fixation primaire de cette dernière, notamment au niveau de la calotte supérieure.

L'objet de l'invention est de s'affranchir de ces différents inconvénients.

Elle propose un cotyle du type en question, facile à réaliser et à mettre en place, et dont la structure favorise outre l'ancrage par fixation primaire, également la repousse osseuse et donc la fixation secondaire, augmentant ainsi de manière significative le maintien de la cupule dans ladite cavité cotyloïdienne.

Ce cotyle pour prothèse de hanche comprend :
- une cupule de soutien destinée à être mise en place dans la cavité cotyloïdienne de l'aile iliaque de l'articulation à prothéser ;
- un insert de frottement destiné :
   ◆ à être inséré dans la cupule de soutien ;
   ◆ à recevoir la tête de l'élément fémoral de ladite prothèse.

Ce cotyle se caractérise :
■ en ce que la base de la surface externe de la cupule de soutien comporte des dents en relief, usinées dans la masse, et s'étendant sensiblement selon des portions de méridien ;
■ et en ce que la surface externe de ladite cupule comporte également au niveau de sa base, trois jeux de deux ailettes dirigés radialement, s'étendant également selon des portions de méridien, mais selon une distance supérieure à la hauteur desdites dents.

En d'autres termes, l'invention consiste à optimiser la fixation primaire de par la mise en oeuvre de ces jeux d'ailettes en coopération avec les dents, qui en outre facilitent la mise en place de la cupule au sein de la cavité cotyloïdienne de par la forme particulière desdites ailettes, et d'autre part, également à optimiser la fixation primaire de par la présence des dents méridiennes.

Selon une caractéristique avantageuse de l'invention, les trois jeux de deux ailettes sont répartis de telle sorte que deux des jeux forment entre eux un angle d'environ 140°, les trois jeux formant en outre deux à deux un angle d'environ 110°, ces différentes valeurs angulaires étant mesurées entre la médiane de chacun des trois jeux. De la sorte, on aboutit à une meilleure implantation de la cupule au sein de la cavité cotyloïdienne, et notamment, l'implantation de deux des jeux au niveau des cornes, respectivement antérieure et postérieure de l'acétabulum.

En outre, la hauteur des ailettes est sensiblement identique à celle des dents.

Par ailleurs, ces ailettes présentent un profil s'amincissant en direction de leurs extrémités radiales, propres à leur conférer un aspect acéré, favorisant ainsi leur insertion ou pénétration au niveau de la partie osseuse de la cavité cotyloïdienne.

Avantageusement, la calotte de la cupule comporte cinq orifices de fixation destinés à permettre le passage de vis pour optimiser la fixation primaire de la cupule au sein de ladite cavité cotyloïdienne.

La cupule présente en outre un orifice fileté, ménagé selon son axe de symétrie au niveau de l'extrémité supérieure de la calotte, destinée à permettre le positionnement de l'ancillaire de pose. Cet orifice fileté est susceptible d'être bouché par un bouchon vissé au niveau dudit filetage, de telle sorte à aboutir à une continuité de la surface de la cupule, et en outre, d'éviter le passage de débris générés par le frottement entre la cupule et l'insert, et dont il a pu être montré qu'il a pu être à l'origine d'ostéolyse, et partant de risques de descellement de la cupule.

La manière dont l'invention peut être réalisé et les avantages qui en découlent ressortiront mieux de l'exemple de réalisation qui suit donné à titre indicatif et non limitatif à l'appui des figures annexées.
La figure 1 est une représentation schématique en perspective d'un cotyle conforme à l'invention, dont la figure 2 est une vue en section transversale.
La figure 3 est une représentation schématique en perspective d'une autre forme de réalisation du cotyle, dont la figure 4 est une vue éclatée, la figure 5 une vue du dessous, et la figure 6 est une vue en section de la cupule représentée à la figure 5.
La figure 7 est une représentation schématique d'un détail de la figure 5.

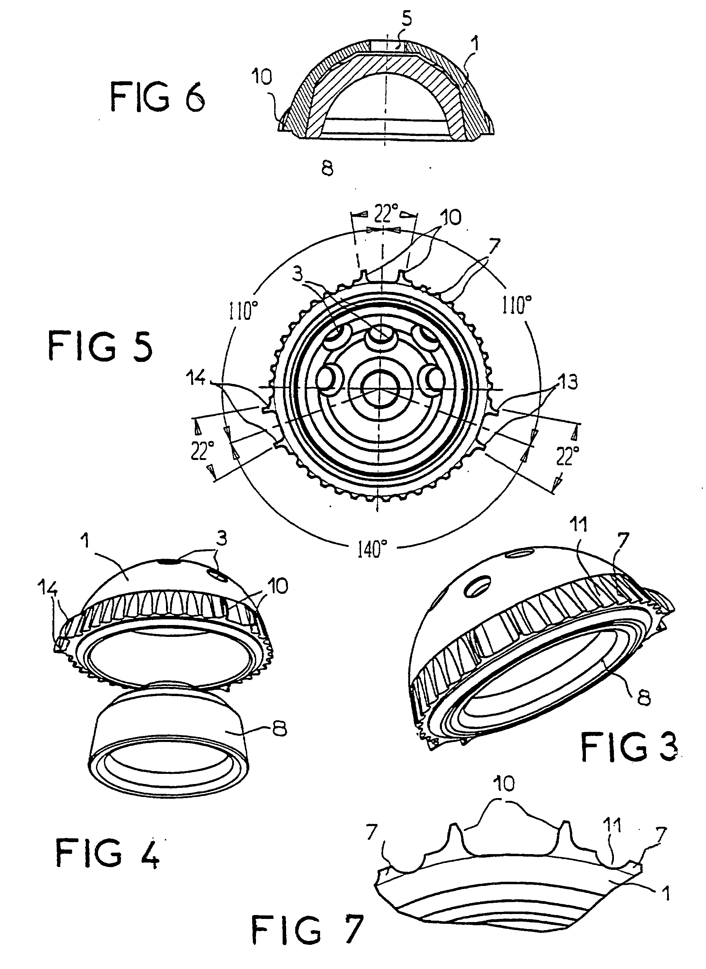

Le cotyle conforme à l'invention est constitué, ainsi que déjà précisé, d'une cupule de soutien désignée sous la référence générale (1), montrée en détail notamment au niveau de la figure 5, et un noyau ou insert (2) ou (8).

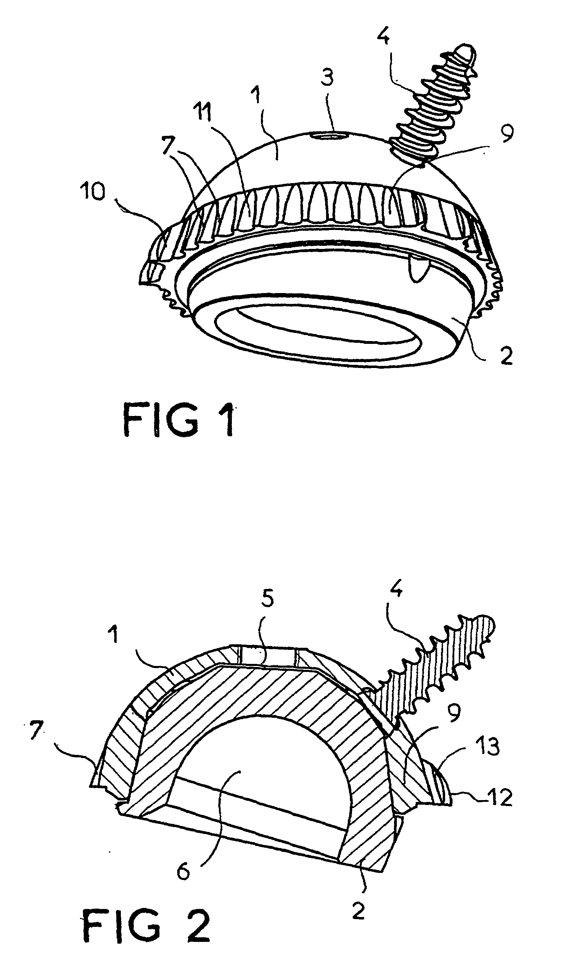

Cet insert est typiquement réalisé en polyéthylène tel que dans les figures 1 et 2, ou en alumine, tel que décrit en liaison avec les figures 3 et 4.

Dans un cas comme dans l'autre, cet insert définit une cavité hémisphérique (6), destiné à coopérer avec la tête sphérique ménagée et rapportée à l'extrémité de l'élément fémoral (non représenté) implanté dans le canal médullaire du fémur à prothéser.

La cupule de soutien conforme à l'invention est réalisée en métal, par exemple en acier inoxydable ou en titane.

Elle présente une face externe convexe, dont la partie supérieure est sensiblement hémisphérique, et se termine au voisinage de son pôle par une ouverture traversante, filetée (5), destinée à permettre l'adaptation d'un ancillaire de pause de cette cupule dans la cavité cotyloïdienne, et notamment d'un impacteur.

Ainsi qu'on peut bien l'observer sur la figure 6, la cupule présente donc une zone légèrement aplatie au niveau du pôle.

Par ailleurs, hormis quelques singularités décrites ci-après plus en détail, cette cupule de soutien présente une symétrie de révolution par rapport à l'axe central passant par le centre de l'orifice (5).

Selon une première caractéristique de l'invention, la cupule présente sur sa surface externe et au niveau de sa base (9) des dents (7), orientées sensiblement radialement selon des portions de méridiens, et obtenues par usinage, et notamment par fraisage dans la masse.

Ainsi qu'on peut l'observer sur les figures, l'épaisseur des dents (7) augmente de la base équatoriale vers leur hauteur maximum. Corollairement, leur profondeur relative diminue.

En effet et ainsi que cela apparaît, les dents (7) ne s'étendent que sur partie seulement de la surface externe de ladite cupule, et typiquement selon une longueur correspondant sensiblement au tiers de la hauteur de ladite cupule.

Avantageusement, l'épaisseur maximum des dents au niveau de la base équatoriale de la cupule, c'est à dire à leur extrémité inférieure est comprise entre 0,7 et 1 mm. Elles sont avantageusement orientées selon un angle de 8° par rapport à l'axe central de révolution de la cupule.

Enfin, la hauteur des dents par rapport à la périphérie de la cupule, et donc la profondeur des rainures (11) ménagées entre celles-ci, est typiquement comprise entre 12 et 15% du diamètre externe maximal de ladite cupule et ce, quelle que soit la taille de la cupule mise en oeuvre.

Selon une autre caractéristique essentielle de l'invention, la surface externe de la cupule présente trois jeux de deux ailettes (10, 13, 14), s'étendant également selon des portions de méridien, selon une longueur identique à celle des dents (7) précédemment décrites, mais présentant une saillie ou excroissance par rapport à l'épure définie par lesdites dents. En effet, la hauteur de ces ailettes, mesurée par rapport à l'enveloppe externe qu'aurait la cupule en l'absence soit des dents, soit des ailettes, est voisine de 2 mm.

De plus, et contrairement aux dents (7), ces ailettes présentent un profil curviligne, dont le bord libre (12) est acéré et affûté, et ce dans le but de favoriser leur pénétration et leur ancrage au niveau de la cavité cotyloïdienne lors de la phase d'impactage, et donc de mise en place de la cupule au sein de cette cavité.

Ainsi qu'on peut bien l'observer sur la figure 5, ces trois jeux de deux ailettes sont répartis de telle sorte que deux des jeux, qualifiés ci-après de jeux inférieurs (13, 14) forment entre eux un angle d'environ 140°, lesdits jeux inférieurs (13, 14) formant en outre avec le jeu dit supérieur (10) un angle d'environ 110°. Ces différentes valeurs angulaires sont mesurées entre la médiane de chacun des trois jeux.

La répartition géographique de ces trois jeux permet ainsi d'aboutir à une implantation des jeux inférieurs (13, 14) au niveau des cornes antérieure et postérieure de l'acétabulum, et partant, d'optimiser la fixation primaire de la cupule.

Cette première singularité constitue donc l'une des exceptions à la symétrie de révolution précédemment mentionnée.

Ces ailettes, outre le fait qu'elle favorise l'ancrage et donc la fixation primaire, joue également un rôle contre la rotation éventuelle de la cupule au sein de la cavité cotyloïdienne.

Selon une autre caractéristique de l'invention, le fond des rainures et de l'espace inter-ailettes est sensiblement tangent avec le diamètre de fraisage de la cupule, ainsi qu'on peut l'observer, par exemple, sur la figure 5. Ce faisant, on évite de la sorte la formation d'un épaulement, et partant, on facilite la mise en place de ladite cupule lors de la phase d'impaction.

Par ailleurs, et avantageusement, toute la cupule est recouverte d'hydroxyapatite, favorisant la fixation secondaire. Ce faisant, et compte-tenu en outre du profil particulier inhérent aux dents (7) et aux rainures inter-dentaires (11), elle favorise également cette repousse osseuse et donc la fixation secondaire qui, combinées avec les moyens participant à la fixation primaire constitués d'une part, par les ailettes et d'autre part, les dents proprement dites, permettent d'aboutir à une fixation très efficace de la cupule au sein de ladite cavité.

Avantageusement, la fixation primaire est en outre optimisée par la mise en oeuvre de vis (4), insérées au niveau d'orifices traversants (3) ménagés au sein de la partie supérieure de la cupule, comme on peut le voir sur les figures 1, 3 et 5.

De fait ces orifices sont regroupés au sein d'une même moitié de l'hémisphère constitutive de la cupule (voir notamment la figure 5), constituant une autre singularité à la symétrie de révolution de la cupule. Plus précisément, la cupule comporte cinq orifices traversants (3), répartis symétriquement par rapport à un plan méridien passant par la médiane du jeu supérieur d'ailettes (10). En fait, trois seulement de ces orifices sont normalement utilisés, pour le passage des vis de fixation, à savoir l'orifice situé sur le plan de symétrie précité, et deux orifices situés de part ou d'autre dudit plan. De la sorte, les vis sont situées dans la partie supéro-postérieure de la cupule, favorisant la fixation primaire.

En outre, de par la symétrie réalisée, tant pour l'implantation des orifices, que pour la répartition des jeux d'ailettes, par rapport au même plan méridien, une même cupule peut donc être implantée dans l'articulation droite ou gauche considérée. Ce faisant, on diminue de moitié la gamme de cupules en termes de taille, diminuant significativement le coût d'un set entier.

Par ailleurs, selon une forme avantageuse de réalisation de l'invention, l'orifice (5) central, permettant la mise en oeuvre de l'ancillaire d'impactage, peut recevoir une bouchon vissable (non représenté), permettant ainsi de générer une continuité tant au niveau de la surface externe que de la surface interne de ladite cupule. Ce faisant, cette continuité s'oppose à l'éventuel transfert des débris susceptibles de survenir de par la coaptation de l'insert (6) avec la cupule (1) au niveau de la zone entre la cupule et l'os de la cavité au niveau de laquelle elle est implantée, et dont il a pu être montré qu'ils étaient susceptibles d'induire une ostéolyse locale, et partant le descellement de ladite cupule.

On conçoit donc l'intérêt d'un tel cotyle, permettant notamment d'augmenter sensiblement la fixation de la cupule au sein de la cavité cotyloïdienne, et d'autre part, facilitant cette mise en oeuvre compte-tenu de la mise en place de ces ailettes qui, de par leur profil acéré, rend plus aisée la pénétration de ladite cupule au sein de la cavité cotyloïdienne préalablement fraisée par le praticien.

## Revendications

1. Cotyle pour prothèse de hanche comprenant :
• une cupule de soutien (1) destinée à être mise en place dans la cavité cotyloïdienne de l'aile iliaque de l'articulation à prothéser ;
• un insert de frottement (2, 8) destiné :
◆ à être inséré dans la cupule de soutien (1) ;
◆ à recevoir la tête de l'élément fémoral de ladite prothèse ;
***caractérisé* :**
■ **en ce que** la base de la surface externe de la cupule de soutien (1) comporte des dents en relief (7), usinées dans la masse, et s'étendant sensiblement selon des portions de méridien ;
■ et **en ce que** la surface externe de ladite cupule (1) comporte également au niveau de sa base, trois jeux de deux ailettes (10, 13, 14) dirigés radialement, s'étendant également selon des portions de méridien, mais selon une distance supérieure à la hauteur desdites dents.

2. Cotyle pour prothèse de hanche selon la revendication 1, ***caractérisé* en ce que** les trois jeux de deux ailettes (10, 13, 14) sont répartis de telle sorte que deux des jeux, dits jeux inférieurs (13, 14) forment entre eux un angle d'environ 140°, les trois jeux formant en outre deux à deux un angle d'environ 110°, ces différentes valeurs angulaires étant mesurées entre la médiane de chacun des trois jeux.

3. Cotyle pour prothèse de hanche selon la revendication 2, ***caractérisé* en ce que** lesdits jeux inférieurs (13, 14) d'ailettes sont ménagés symétriquement l'un de l'autre par rapport à un plan méridien passant par la médiane du jeu supérieur d'ailettes (10).

4. Cotyle pour prothèse de hanche selon l'une des revendications 1 à 3, ***caractérisé* en ce que** la longueur des ailettes des jeux (10, 13, 14) est sensiblement identique à celle des dents (7).

5. Cotyle pour prothèse de hanche selon la revendication 4, ***caractérisé* en ce que** les ailettes des jeux (10, 13, 14) et les dents (7) s'étendent selon une longueur correspondant sensiblement au tiers de la hauteur de la cupule de soutien (1).

6. Cotyle pour prothèse de hanche selon l'une des revendications 1 à 5, ***caractérisé* en ce que** les ailettes des jeux (10, 13, 14) présentent un profil curviligne qui s'amincit en direction de leur bord libre (12), propre à leur conférer un aspect acéré, favorisant ainsi leur insertion ou pénétration au niveau de la partie osseuse de la cavité cotyloïdienne.

7. Cotyle pour prothèse de hanche selon l'une des revendications 1 à 6, ***caractérisé* en ce que** la calotte de la cupule de soutien (1) comporte des orifices de fixation (3) destinés à permettre le passage de vis (4) pour optimiser la fixation primaire de la cupule au sein de ladite cavité cotyloïdienne.

8. Cotyle pour prothèse de hanche selon la revendication 7, ***caractérisé* en ce que** les orifices sont au nombre de cinq, répartis de manière symétrique par rapport au plan méridien passant par la médiane du jeu supérieur (10) d'ailettes.

9. Cotyle pour prothèse de hanche selon l'une des revendications 1 à 8, ***caractérisé* en ce que** le fond des rainures (11) définies par les dents (7) et de l'espace inter-ailettes est sensiblement tangent avec le diamètre de fraisage de la cupule.

10. Cotyle pour prothèse de hanche selon l'une des revendications 1 à 9, ***caractérisé* en ce que** la cupule de soutien présente en outre un orifice central fileté (5), ménagé selon son axe de symétrie au niveau de l'extrémité supérieure de la calotte, destinée à permettre le positionnement de l'ancillaire de pose.

11. Cotyle pour prothèse de hanche selon la revendication 10, ***caractérisé* en ce que** l'orifice fileté (5) est susceptible d'être bouché par un bouchon vissé au niveau dudit filetage, de telle sorte à aboutir à une continuité de la cupule, et en outre, d'éviter le passage de débris liés générés par le frottement entre la cupule et l'insert (2, 8),

## Patentansprüche

1. Gelenkpfanne für eine Hüftprothese, welche folgendes aufweist:
• eine Haltehaube (1), die dazu bestimmt ist, in die Gelenkshöhlung der Beckenschaufel des zu ersetzenden Gelenks eingesetzt zu werden;
• ein Reibeinsatz (2, 8), der dazu bestimmt ist:
◆ in die Haltehaube (1) eingesetzt zu werden; und
◆ den Kopf des femoralen Elementes der Prothese aufzunehmen;
***dadurch gekennzeichnet,***
■ **daß** die Basis der Außenfläche der Haltehaube (1) erhabene, in die Masse eingearbeitete Zähne (7) aufweist, die sich im wesentlichen entlang von Meridianteilen erstrecken;
■ und **daß** die Außenfläche der Haube (1) in der Höhe ihrer Basis auch drei Sätze von je zwei radial ausgerichteten Flügeln (10, 13, 14) aufweist, die sich ebenfalls entlang von Meridianteilen erstrecken, aber über eine gegenüber der Höhe der Zähne größeren Distanz.

2. Gelenkpfanne für eine Hüftprothese nach Anspruch 1, ***dadurch gekennzeichnet,* daß** die drei Sätze von je zwei Flügeln (10, 13, 14) derart verteilt sind, daß zwei der Sätze, die sogenannten unteren Sätze (13, 14) miteinander einen Winkel von ungefähr 140° bilden, wogegen von den drei Sätzen zwei jeweils einen Winkel von etwa 110° einschließen, wobei die verschiedenen Winkelwerte zwischen den Mitten jedes der drei Sätze gemessen sind.

3. Gelenkpfanne für eine Hüftprothese nach Anspruch 2, ***dadurch gekennzeichnet,* daß** die beiden unteren Sätze (13, 14) von Flügeln jeweils symmetrisch bezüglich einer Meridianebene ausgebildet sind, die durch die Mitte des oberen Satzes von Flügeln (10) verläuft.

4. Gelenkpfanne für eine Hüftprothese nach einem der Ansprüche 1 bis 3, ***dadurch gekennzeichnet,* daß** die Länge der Flügeln der Sätze (10, 13, 14) mit jener der Zähne (7) im wesentlichen identisch ist.

5. Gelenkpfanne für eine Hüftprothese nach Anspruch 4, ***dadurch gekennzeichnet,* daß** die Flügel der Sätze (10, 13, 14) und die Zähne (7) sich über eine Länge erstrecken, welche im wesentlichen einem Drittel der Höhe der Haltehaube (1) entspricht.

6. Gelenkpfanne für eine Hüftprothese nach einem der Ansprüche 1 bis 5, ***dadurch gekennzeichnet,* daß** die Flügel der Sätze (10, 13, 14) ein gekrümmtes Profil aufweisen, welches in Richtung auf ihren freien Rand (12) schmäler wird, so daß sie ein spitzes Aussehen erhalten, welches so ihr Einsetzen bzw. Eindringen in der Höhe des Knochenteiles der Gelenkshöhlung begünstigt.

7. Gelenkpfanne für eine Hüftprothese nach einem der Ansprüche 1 bis 6, ***dadurch gekennzeichnet,* daß** die Kalotte der Haltehaube (1) Fixieröffnungen (3) aufweist, welche dazu bestimmt sind, den Durchtritt einer Schraube (4) zu gestatten, um die primäre Fixierung der Haube im Inneren der Gelenkshöhlung zu optimieren.

8. Gelenkpfanne für eine Hüftprothese nach Anspruch 7, ***dadurch gekennzeichnet,* daß** die Öffnungen in einer Anzahl von fünf vorhanden sind, die symmetrisch bezüglich einer Meridianebene angeordnet sind, welche durch die Mitte des oberen Satzes von Flügeln (10) verläuft.

9. Gelenkpfanne für eine Hüftprothese nach einem der Ansprüche 1 bis 8, ***dadurch gekennzeichnet,* daß** der Boden der durch die Zähne (7) begrenzten Nuten (11) sowie des Zwischenraumes zwischen den Flügeln im wesentlichen tangential zum Fräsdurchmesser der Haube verläuft.

10. Gelenkpfanne für eine Hüftprothese nach einem der Ansprüche 1 bis 9, ***dadurch gekennzeichnet,* daß** die Haltehaube überdies eine zentrale Gewindeöffnung (5) aufweist, die auf der Höhe des obersten Endes der Kalotte in der Symmetrieachse eingebracht und zur Positionierung einer Einbauhilfe bestimmt ist.

11. Gelenkpfanne für eine Hüftprothese nach Anspruch 10, ***dadurch gekennzeichnet,* daß** die Gewindeöffnung (5) durch einen Schraubpfropfen in Höhe des Gewindes derart verschließbar ist, daß er an den stetigen Verlauf der Haube angrenzt und überdies den Durchlaß von durch die Reibung zwischen der Haube und dem Einsatz (2, 8) erzeugten zusammenhängenden Abrieb verhindert.

## Claims

1. Acetabular cup for hip prosthesis, comprising:
- a support cup (1) intended to be placed in the acetabular cavity of the iliac crest of the joint to be fitted with the prosthesis;
- a friction insert (2, 8) intended:
• to be inserted into the support cup (1);
• to receive the head of the femoral element of said prosthesis;
**characterized:**
- **in that** the base of the outer surface of the support cup (1) has raised teeth (7) which are machined from the material and extend substantially along meridian portions;
- and **in that** the outer surface of said cup (1) also comprises, in the area of its base, three sets of two radially directed fins (10, 13, 14) which also extend along meridian portions, but by a distance greater than the height of said teeth.

2. Acetabular cup for hip prosthesis according to Claim 1, **characterized in that** the three sets of two fins (10, 13, 14) are distributed in such a way that two of the sets, called the lower sets (13, 14), between them form an angle of about 140°, the three sets additionally forming in pairs an angle of about 110°, these different angular values being measured between the median of each of the three sets.

3. Acetabular cup for hip prosthesis according to Claim 2, **characterized in that** said lower sets (13, 14) of fins are arranged symmetrically to one another in relation to a meridian plane passing through the median of the upper set of fins (10).

4. Acetabular cup for hip prosthesis according to one of Claims 1 to 3, **characterized in that** the length of the fins of the sets (10, 13, 14) is substantially identical to that of the teeth (7).

5. Acetabular cup for hip prosthesis according to Claim 4, **characterized in that** the fins of the sets (10, 13, 14) and the teeth (7) extend by a length corresponding substantially to a third of the height of the support cup (1).

6. Acetabular cup for hip prosthesis according to one of Claims 1 to 5, **characterized in that** the fins of the sets (10, 13, 14) have a curvilinear profile which becomes thinner in the direction of their free edge (12), so as to give them a sharp appearance, thereby promoting their insertion or penetration in the osseous part of the acetabular cavity.

7. Acetabular cup for hip prosthesis according to one of Claims 1 to 6, **characterized in that** the cap area of the support cup (1) comprises fixation orifices (3) intended to permit the passage of screws (4) for optimizing the primary fixation of the cup in said acetabular cavity.

8. Acetabular cup for hip prosthesis according to Claim 7, **characterized in that** the orifices are five in number and are distributed symmetrically in relation to the meridian plane passing through the median of the upper set (10) of fins.

9. Acetabular cup for hip prosthesis according to one of Claims 1 to 8, **characterized in that** the bottom of the grooves (11) defined by the teeth (7) and of the space between the fins is substantially tangential with the milling diameter of the cup.

10. Acetabular cup for hip prosthesis according to one of Claims 1 to 9, **characterized in that** the support cup additionally has a threaded central orifice (5) formed on its axis of symmetry at the upper end of the cap area and intended to permit positioning of the ancillary implantation device.

11. Acetabular cup for hip prosthesis according to Claim 10, **characterized in that** the threaded orifice (5) can be plugged by a stopper which is screwed into said thread, in such a way as to achieve a continuity of the cup and in addition avoid the passage of associated debris generated by the friction between the cup and the insert (2, 8) .
